# EUROPEAN PATENT SPECIFICATION

(11) **EP 2 508 622 B1**
(45) Date of publication and mention of the grant of the patent: **14.09.2016**
(21) Application number: 12159372.7
(22) Date of filing: 22.12.2006
(51) Int. Cl.: C12Q 1/68, C12M 3/00

(54) **Comparative genomic hybridization on encoded multiplex particles**
Vergleichende genomische Hybridisierung auf codierten Multiplexteilchen
Hybridation génomique comparative sur des particules multiplexées codées

(30) Priority: 23.12.2005 US 753584 P; 23.12.2005 US 753822 P; 03.02.2006 US 765311 P; 03.02.2006 US 765355 P
(43) Date of publication of application: 10.10.2012
(62) Divisional of application: 06845950.2
(73) Proprietor: Perkinelmer Las, Inc., Boston, MA 02118 (US)
(72) Inventor: Borrow, Mark N., Lexington, MA 02421 (US); Adler, Karl Edwin, Newburyport, MA 01950 (US); Schermer, Mack J., Belmont, MA 02478 (US)
(74) Representative: Williams, Gareth Owen

(56) References cited:
- WO-A1-96/17958
- WO-A2-03/054149
- WO-A2-2007/022530
- NEWKIRK HEATHER L ET AL: "Determination of genomic copy number with quantitative microsphere hybridization", HUMAN MUTATION, JOHN WILEY & SONS, INC, US, vol. 27, no. 4, 1 April 2006 (2006-04-01), pages 376-386, XP002544643, ISSN: 1059-7794
- NEWKIRK H.L. ET AL: "Genomic Copy Number Detection with Microsphere-Based Suspension Hybridization", ASHG ANNUAL MEETING 2005, ABSTRACT 1273/F , 25 October 2005 (2005-10-25), 29 October 2005 (2005-10-29), XP002553963, Salt Lake City, UTAH, USA Retrieved from the Internet: URL:http://www.ashg.org/cgi-bin/ashg05s/as hg05?pgmnr=1273&author=Newkirk&sort=pgmnum s&sbutton=Detail&absno=1273&sid=214277 [retrieved on 2009-11-05]
- FUJA TANNIN ET AL: "A multiplex microsphere bead assay for comparative RNA expression analysis using flow cytometry", JOURNAL OF BIOTECHNOLOGY, ELSEVIER SCIENCE PUBLISHERS, AMSTERDAM, NL, vol. 108, no. 3, 18 March 2004 (2004-03-18), pages 193-205, XP002412741, ISSN: 0168-1656
- XU HONGXIA ET AL: "Multiplexed SNP genotyping using the Qbead system: a quantum dot-encoded microsphere-based assay", NUCLEIC ACIDS RESEARCH, OXFORD UNIVERSITY PRESS, SURREY, GB, vol. 31, no. 8, 15 April 2003 (2003-04-15) , page e43, XP002308425, ISSN: 0305-1048
- ROGAN P K ET AL: "Sequence-based design of single-copy genomic DNA probes for fluorescence in situ hybridization", GENOME RESEARCH, COLD SPRING HARBOR LABORATORY PRESS, WOODBURY, NY, US, vol. 11, no. 6, 1 June 2001 (2001-06-01), pages 1086-1094, XP002397526, ISSN: 1088-9051, DOI: 10.1101/GR.171701
- KNIJNENBURG JEROEN ET AL: "Rapid detection of genomic imbalances using micro-arrays consisting of pooled BACs covering all human chromosome arms", NUCLEIC ACIDS RESEARCH, OXFORD UNIVERSITY PRESS, GB, vol. 33, no. 18, 1 January 2005 (2005-01-01), pages E159-1, XP002613368, ISSN: 1362-4962, DOI: 10.1093/NAR/GNI161 [retrieved on 2005-10-12]
- FIEGLER H ET AL: "DNA MICROARRAYS FOR COMPARATIVE GENOMIC HYBRIDIZATION BASED DOP-PCR AMPLIFICATION OF BAC AND PAC CLONES", GENES, CHROMOSOMES & CANCER, XX, XX, vol. 36, no. 4, 1 April 2003 (2003-04-01), pages 361-374, XP008040411, DOI: 10.1002/GCC.10155

## Description

### CROSS-REFERENCE TO RELATED APPLICATIONS

This application claims priority to U.S. Application Serial No. 60/753,584, filed on December 23, 2005, U.S. Application Serial No. 60/753,822, filed on December 23, 2005, U.S. Application Serial No. 60/765,311, filed on February 3, 2006, and U.S. Application Serial No. 60/765,355, filed on February 3, 2006.

### BACKGROUND

Comparative Genomic Hybridization (CGH) is a method that evaluates genomic content. CGH can be used to analyze congenital abnormalities, inherited diseases and cancers, for example.

### SUMMARY

We have discovered methods of evaluating genomic content using encoded particles to evaluate multiple samples in parallel. In some embodiments, differences in genomic content can be detected by evaluating DNA from an unknown sample and reference DNA in parallel, e.g., without combining DNA for analysis and reference DNA into a single mixture. The DNA for analysis and reference DNA remain separate from one another. The multiple samples can be evaluated with a single label, for example. In some other aspects described herein, a single detection label is used to detect differences in genomic content by comparing DNA for analysis and reference DNA in the same mixture. The DNA for analysis and the reference DNA can have different direct or indirect labels, for example. The methods can also be used to evaluate other nucleic acids, e.g., mRNA, as in gene expression analysis.

In one aspect, the invention provides a method of evaluating genomic DNA, the method comprising:
providing a plurality of DNA samples, wherein at least one sample of the plurality of samples is a reference sample, with a known number of copies for each interrogated genomic locus, and at least one sample is a genomic DNA sample;
providing a particle mixture, the mixture comprising particles from different particle sets, wherein each particle set contains numerous encoded particles, wherein each particle within a particle set comprises i) the same code, and ii) a nucleic acid hybridization probe for a particular genomic locus, wherein each different particle set is specific for a different genomic locus, such that the mixture collectively includes probes for a plurality of different genomic loci and wherein the nucleic acid hybridisation probe of each particle comprises BAC nucleic acid, wherein the hybridization probe of each particle is produced by sonicating or fragmenting the BAC DNA
labelling the reference sample with a first indirect label and labelling the genomic DNA sample and other samples of the plurality of samples with a second indirect label;
contacting each of the labelled samples to a portion of the particle mixture under hybridization conditions; and
evaluating hybridization of each of the samples to particles in the respective portion of the mixture by monitoring the first indirect label or the second indirect label, or both, wherein signals from monitoring the reference sample are compared to signals from other samples to determine the number of copies of each interrogated genomic locus for the samples.

In one aspect there is described a method of evaluating genomic DNA. The method uses a mixture that includes particles from different particle sets. Each particle set contains numerous encoded particles and a nucleic acid hybridization probe for a particular genomic locus, such that the mixture collectively includes probes for a plurality of different genomic loci. The method can include: providing a genomic DNA sample and a particle mixture; contacting the sample to a portion of the particle mixture under hybridization conditions; and evaluating hybridization of the sample to particles in the respective portion of the mixture by monitoring a detectable label. Signals from the monitoring are indicative of the number of copies of each interrogated genomic locus.

More than one nucleic acid sample (e.g., a sample including genomic DNA) can be provided. For example, each nucleic acid sample can be evaluated according to the method. The samples can be evaluated in separate compartments. In some aspects, the more than one DNA samples can be evaluated in the same compartment.

In some aspects, the detectable label can be detectable by spectroscopy. An exemplary detectable label can contain phycoerythrin or other fluorescent molecule.

In some embodiments, the nucleic acid hybridization probe includes or is derived from cloned nucleic acid. For example, the nucleic acid hybridization probe can contain nucleic acid from a bacterial artificial chromosome (BAC). The BAC nucleic acid can contain a segment of human genomic DNA or non-human (e.g., mouse, rat, rabbit, ginea pig, hamster, goat, cow, dog, cat, horse, bird, reptile, non-human primate (e.g., monkey or baboon), or fly) genomic DNA.

In some embodiments, the nucleic acid hybridization probe can contain one or more oligonucleotides (e.g., a collection of oligonucleotides) specific for a particular chromosomal locus. For example the probes can be specific for sequences that are within 50, 20, 5, 2, 1, or 0.5 megabases of one another.

In some aspects described herein, at least 2, 5, 10, 20, 50, 100, or 200 different particle sets can be used, e.g., to evaluate a respective number of different genomic loci. In some embodiments, all the encoded particles of a particle set can have the same code. In other aspects described herein, each particle has a unique code, and information is stored indicating which particles are in which particle sets or which probes are attached to each particle.

In some embodiments, at least one sample of the plurality can be a reference sample, with a known number of copies for each interrogated genomic locus. The method can include comparing signals from monitoring the reference sample to signals from other samples to determine the number of copies of each interrogated genomic locus for the samples.

In some embodiments, where more than one DNA sample is provided, each sample can be labeled with a first indirect label, and each sample can be combined with reference DNA labeled with a second indirect label. The reference DNA can be genomic DNA from a reference source with a known number of copies for each interrogated genomic locus. For example, the first indirect label can be fluorescein and the second indirect label can be biotin.

In some embodiments, the evaluating can involve (i) binding, to a first portion of the sample, a first moiety that includes the single label and an agent that binds the first indirect label, and (ii) binding, to a second portion of the sample, a second moiety that includes the single label and an agent that binds the second indirect label. For example, the first moiety can include streptavidin or avidin and a detectable label (e.g., phycoerythrin), and the second moiety can include an anti-fluorescein antibody or functional portion thereof and a detectable label (e.g., phycoerythrin). In some aspects described herein, at least 5, 10, 20, 30, 50, 70, or 100 particles from each of the different particle sets for each genomic sample are evaluated.

Where more than one sample is provided, each of the more than one samples can be in a different compartment of a multi-compartment device. In some aspects described herein, each sample can be in a different well of a multi-well plate. The multi-well plate can be a 96-well, 384-well, or 1024-well assay plate.

The method can be used, e.g., to detect heterozygosity at a plurality of different chromosomal loci, chromosomal amplification can be detectable, loss of heterozygosity, , a heterozygous deletion of a chromosomal locus, or a homozygous deletion of a chromosomal locus.

In some aspects described herein, the particles are not contacted with a polymerase, e.g., after the hybridization. In some aspects, the particles are not contacted with any enzyme, e.g., after the hybridization.

As also described herein, the genomic DNA samples can be unlabeled. The method can further include hybridizing labeled probes to the genomic samples, wherein, for each of the nucleic acid hybridization probe attached to particles, a labeled probe hybridizes to a genetically linked site at the same genomic locus, such that if the genomic locus is present in the sample, the labeled probe can be immobilized to the particle by a complex formed by hybridization of the labeled probe to a sample nucleic acid strand and hybridization of the sample nucleic acid strand to the nucleic acid hybridization probe attached to the particle. In some aspects, the labeled probes can be hybridized concurrently with hybridizing the genomic DNA samples to the nucleic acid probes attached to the particles. In some aspects, the labeled probes can be hybridized subsequent to hybridizing the genomic DNA samples to the nucleic acid probes attached to the particles.

In some embodiments, the method can further include labeling genomic DNA from a source with an indirect label to provide a genomic DNA sample.

Where a single detectable label is used, the method can further include the step of labeling genomic DNA from a source with the single label to provide a genomic DNA sample. In some embodiments of the method, all of the genomic DNA samples can be labeled with the single label.

In some aspects described herein, all of the genomic DNA samples having unknown genomic content can be labeled with the same indirect label. In some aspects, a majority of the genomic DNA samples can be labeled with the same indirect label.

In some embodiments, the method can further include agitating the particles prior to evaluating hybridization.

For example, the particles can be holographically encoded or encoded with fluorescent dyes that have spectra separable from that of the single detected label. In another aspect described herein, the particles can have magnetic properties. For example, the particles are paramagnetic beads.

In another aspect, we describe a method of evaluating genomic DNA using a single detectable moiety and a particle mixture, the mixture including particles from different particle sets, wherein each particle set contains numerous encoded particles and a nucleic acid hybridization probe for a particular genomic locus, such that the mixture collectively includes probes for a plurality of different genomic loci. The method includes: providing at least one reference DNA sample and a plurality of genomic DNA samples. Each sample is labeled with the same detectable moiety. The method also includes: contacting each of the samples to a portion of the particle mixture under hybridization conditions; and evaluating hybridization of each sample to particles in the respective portion of the mixture by monitoring the detectable moiety. For example, each sample is contacted to the particle mixture in a separate vessel. The signals from the monitoring can be indicative of the number of copies of each interrogated genomic locus. The method can include other features described herein.

In another aspect, the disclosure features a method of evaluating nucleic acid using a single detectable label and a particle mixture, the mixture including particles from different particle sets, wherein each particle set contains numerous encoded particles and a nucleic acid hybridization probe for a particular target, such that the mixture collectively includes probes for a plurality of different target. The method can include: providing at least one reference nucleic acid sample that is labeled with a first indirect label and a plurality of test nucleic acid samples. Each test sample is labeled with a second indirect label. The method can include: contacting each of the test samples and the reference sample to a portion of the particle mixture under hybridization conditions, wherein each test sample is contacted to the particle mixture and the reference sample in a separate vessel; binding, to a first portion of each test sample, a first moiety that includes the single label and an agent that binds the first indirect label; binding, to a second portion of each test sample, a second moiety that includes the single label and an agent that binds the second indirect label; evaluating each test sample by monitoring the single label in the first portion of the sample and the single label in the second portion of the sample. The signals from the monitoring can be indicative of the number of copies of each target. The method can include, for each test sample, comparing signals from the single label in the first portion to signals from the single label in the second portion, to obtain an indication of the number of copies of a probe in the test sample relative to the reference sample. The method can include other features described herein.

In another aspect, we describe a particle mixture, which mixture contains particles from different particle sets, wherein each particle set can contain numerous encoded particles and a nucleic acid hybridization probe for a particular genomic locus, such that the mixture collectively includes probes for a plurality of different genomic loci.

In some easpects described herein, the probe for at least some of the loci can contain bacterial artificial chromosome DNA. In some embodiments, the probe for at least some of the loci can contain sonicated bacterial artificial chromosome DNA. In some aspects, the probe for at least some of the loci can contain a collection of synthetic oligonucleotides.

In some aspects, the particle mixture can further include particles including hybridized DNA from at least two samples, wherein each sample includes genomic DNA and each sample can be labeled with a different indirect label. In some aspects, the particle mixture can further include hybridized DNA from a single sample that is labeled with a detectable label, or hybridized DNA from more than a single sample (e.g., a first sample and a second sample)..

In another aspect, we describe a multi-compartment plate having a multiple compartments, where each of at least a plurality of the wells can contain: a particle mixture such as any of those described herein; and a sample or reference genomic nucleic acid. The sample and the reference nucleic acid can be in separate vessels. In one aspect, the sample and reference nucleic acid can be detectable with the same label.

In another aspect, the disclosure feature a kit that includes: a reference genomic DNA (or other reference nucleic acid) sample labeled with a first indirect label; reagents for labeling genomic DNA (or other nucleic acid) samples with a second indirect label. The kit can also include one or more of: a first moiety that includes the single label and an agent that binds the first indirect label; a second moiety that includes the single label and an agent that binds the second indirect label; and a particle mixture such as any of those described herein. The kit can also include, optionally, instructions for how to detect the single label and/or for performing an assay using the kit.

Also disclosed is a kit that includes one or more of the components described herein. The kit can further include materials that includes instructions for using the components for a method, e.g., a method described herein. The kit can include one or more cloned or synthesized nucleic acid sequences (such as bacterial artificial chromosomes (BACS) or one or more collections of synthetic oligonucleotides, or individual oligonucleotides), one or more microwell plates, and/or one or more sets of particles, e.g., as described herein, or a mixture of particles.

Particles, such as beads, provide a particularly robust system for evaluating genomic content. Particle-base methods include hybridization close to or at solution phase kinetics, therefore providing enhanced uniformity. Particles facilitate obtaining many data points for a particular probe. For example, for small particles (e.g., particles up to several microns in diameter) at least 10, 50, or 100 particles having the same probe can be individually analyzed. Large particles can enable obtaining multiple data points from discreet locations on each particle. For large particles, often fewer particles are needed to obtain many data points. Statistics can be applied to determine the median or average signal for the population of particles with the same probe content.

The methods described herein can be used for ratiometric assays. The ratiometric approach corrects for many potential errors in the accuracy of the assay by performing multiple assays competitively and simultaneously. Any variations in incubation conditions or the concentrations of the capture molecules or label reagents, for example, are corrected by normalization. Competitive and non-competitive ratiometric assays can be used, e.g., for comparative genomic content or gene expression.

### BRIEF DESCRIPTION OF THE DRAWINGS

FIGS. 1-6 are schematic diagrams of a series of specific binding interactions with capture molecules immobilized on assay beads according to one example.
FIG. 7 is a process flow chart of the example assay described in FIGS. 1 through 6.
FIGS. 8-10 depict results of reference assays performed according to exemplary embodiments.
FIG. 11 depicts results from an exemplary assay using dual indirect label-single hybridization for expression RNA.
FIG. 12 is a line graph depicting results from a single-color (single label) assay performed according to one embodiment, where reference and test samples were not in same well.

### DETAILED DESCRIPTION

Multiplex nucleic acid assays are greatly facilitated by efficient and economical design features. As described herein, single-label detection can be used to perform large multiplex assays. The assays utilizes a mixture of small particles from different particle sets. Each particle set contains numerous encoded particles and a nucleic acid hybridization probe for a particular target, such as a particular genomic locus or RNA. Particles from different sets can be combined to provide a mixture that collectively includes probes for a plurality of different targets.

In one example, the particle mixture is divided into at least two portions, A and B. The test sample genomic DNA is labeled with a first moiety and hybridized to the labeled A portion. The reference DNA (typically genomic DNA from a reference sample) is labeled with a moiety that can be the same as the first moiety or different (i.e., a second moiety). The reference DNA is hybridized to the B portion. The signals from the A portion and the B portion are measured and then compared. If the reference DNA and the sample DNA are labeled with the same moiety, they are processed in different vessels. With little manipulation, both hybridization reactions can be evaluated using a device configured to detect the moiety if it is a direct label or is coupled to a direct label (e.g., before or after hybridization).

The moieties that are used can be an indirect label (such as a dye used as an indirect label), a direct label (such as dye, e.g. a fluorophore), or a member of a specific binding pair. Detection can be direct or indirect, e.g., by secondary detection by using a labeled second member of the specific binding pair.

In a second example, the test and reference sample genomic DNA are labeled, each with a different indirect label. The indirect label is typically a first member of a specific binding pair. The samples are mixed with each other and hybridized to the same portion of a particle mixture. After incubation and washing, the portion is separated into at least two vessels for incubation with the corresponding second members of the two specific binding pairs. These second members of the two specific binding pairs can be coupled to a third label, and if needed a fourth label, which is used for detection. Generally, the detector label and the two indirect labels are all distinguishable from one another. For example, if one or more of the labels are fluorescent labels, they have different excitation and/or emission spectra so that they can be distinguished by spectroscopy.

To illustrate, biotin can be used as the indirect label for the test sample and carboxyfluorescein as the indirect label for the reference sample. Thus, in the first vessel, fluorescently labeled streptavidin (which binds to the biotin) is added and incubated, and in the second, fluorescently labeled anti-fluorescein (which binds to the carboxyfluorescein) is added and incubated. For the LUMINEX xMAP™ system the preferred fluorescent label for detection is phycoerythrin. Each of the incubated particle mixtures with bound fluorescently labeled products (from the test and reference sample) are then read sequentially by using a single-color detection system and the signals compared.

Both these examples illustrate methods for using a single detection to obtain comparative or ratiometric information. In an exemplary application, numerous different patient samples can be processed in a single multiwell plate using a mixture of particles with probes for different targets, e.g., different genomic loci. The particle mixture is placed in each well of the plate. Each sample is put in one of the wells. In this fashion, multiple samples can be processed in parallel and with robotic aids. Then each sample can be evaluated using an instrument (such as a flow cytometer) in order to detect hybridization to the different particle types in the mixture.

In some aspects described herein, the test and reference samples are labeled with two different direct or indirect labels. If the labels are direct (e.g., fluorescent dyes such as cyanine 3 and cyanine 5), then the samples may be mixed and hybridized to a particle set and detected with an instrument capable of discriminating the two labels. If the labels are indirect (i.e., a first member of a specific binding pair such as biotin and fluorescein) then each second member of a specific binding pair (e.g., streptavidin and anti-fluorescein) can be labeled with a unique detector label such as cyanine 3 and cyanine 5, and detected with an instrument capable of discriminating the two labels.

### Particles

A variety of different types of particles can be used to evaluate nucleic acid content, e.g., genomic content. The particles can be of any shape (e.g., cylindrical, spherical, and so forth), size, composition, or physiochemical characteristics. The particle size or composition can be chosen so that the particle can be separated from fluid, e.g., on a filter with a particular pore size or by some other physical property, e.g., a magnetic property.

The particles are generally suitable for multiplex assays. For example, each particle includes a unique code, particularly, a code other than the nucleic acid probe specific for genomic DNA. The code is embedded (for example, within the interior of the particle) or otherwise attached to the particle in a manner that is stable through hybridization and analysis. The code can be provided by any detectable means, such as by holographic encoding, by a fluorescence property, color, shape, size, light emission, quantum dot emission and the like to identify particle and thus the capture probes immobilized thereto. In some aspects, the code is other than one provided by a nucleic acid.

For example, the particles may be encoded using optical, chemical, physical, or electronic tags. Examples of such coding technologies are optical bar codes fluorescent dyes, or other means.

One exemplary platform utilizes mixtures of fluorescent dyes impregnated into polymer particles as the means to identify each member of a particle set to which a specific capture probe has been immobilized. Another exemplary platform uses holographic barcodes to identify cylindrical glass particles. For example, Chandler et al. (5,981,180) describes a particle-based system in which different particle types are encoded by mixtures of various proportions of two or more fluorescent dyes impregnated into polymer particles. Soini (5,028,545) describes a particle-based multiplexed assay system that employs time-resolved fluorescence for particle identification. Fulwyler (4,499,052) describes an exemplary method for using particle distinguished by color and/or size. US 2004-0179267, 2004-0132205, 2004-0130786, 2004-0130761, 2004-0126875, 2004-0125424, and 2004-0075907 describe exemplary particles encoded by holographic barcodes.

US 6,916,661 describes polymeric microparticles that are associated with nanoparticles that have dyes that provide a code for the particles. The polymeric microparticles can have a diameter of less than one millimeter, e.g., a size ranging from about 0.1 to about 1,000 micrometers in diameter, e.g., 3-25 µm or about 6-12 µm. The nanoparticles can have, e.g., a diameter from about 1 nanometer (nm) to about 100,000 nm in diameter, e.g., about 10 - 1,000 nm or 200 - 500 nm.

Newkirk et al., 29.10.2005 discloses a microsphere-based suspension hybridization assay that has been developed for high throughput detection of changes in genomic copy number. A particle mixture for examining multiple genomic loci can be prepared by combining particles from different particle sets. Each particle set can contain numerous particles with the same code and nucleic acid probe content specific for the particular locus. For example, the number of different particle elements can be in the range of 10 - 3000, 10 - 500, 20-200, 50 - 2000, 50 - 500, or 50 - 200, or 75 - 300. Each particle set can be prepared individually, then a portion of the particle set is combined with portions of other particle sets to provide a particle mixture.

Each portion of a particle set includes numerous particles such that when a portion of the particle mixture is used for analyzing a DNA sample, a number of particles specific for a particular locus (for example, at least 10, 20, 30, 50, 80, or 100 particles; or even 1-5) are present in the hybridization reaction for that individual sample. The particles can be agitated in suspension within a liquid sample to facilitate relatively rapid binding to the probes.

Particles are analyzed to determine the extent of hybridization to the particles. For example, a label associated with the genomic DNA is detected. The particles can be individually evaluated using a device that can read the particle code and determine signals associated with each particular particle.

### Nucleic Acid Probes

The particles generally have probes specific to one particular chromosomal locus or gene, depending on the application. For example, probes can be prepared from cloned DNA (for example, BAC or Yeast Artificial Chromosome or other source cloned DNA), amplified DNA, or oligonucleotides. Generally, any source of nucleic acid of determinable sequence can be used. The probes are immobilized to the particles, e.g., on the surface or for porous particles or to internal and/or external surfaces.

One chromosomal probe comprises a mixture of synthesized oligonucleotides. Another exemplary type of chromosomal probe is derived from BAC cloned DNA which can be immobilized as fragmented BAC DNA.

Generally, the probes that can be attached for a particular particle can be a mixture of fragments. For example, the fragments comprise randomly overlapping sequences, the majority of which have lengths between 600 and 2,000 base pairs. A mixture of synthetic oligonucleotide sequences, each of which having a length between about 60 and 150 base pairs, with either overlapping or contiguous sequences, can be prepared as a probe. Oligonucleotide mixture have a deterministic composition and can be prepared in a cost efficient manner.

The probes can be covalently attached to a particular particle. For example, US 6,048,695 describes an exemplary method for attaching probes. If the probe for a particular particle set is one or more chemically synthesized oligonucleotides, the oligonucleotides can be synthesized with a chemical group (e.g., an amine) which can react with groups on the particle. The probe can also be derived from other sources, e.g., plasmids, cosmids, and artificial chromosomes. For example, a bacterial artificial chromosome (BAC) can be sonicated then reacted with a crosslinker that covalently attaches them to the particles. For example, 1-Ethyl-3-[3-dimethylaminopropyl]carbodiimide hydrochloride (EDC or EDAC chemistry) can be used to attach nucleic acid probes, such as sonicated BAC DNA to particles.

Generally, when attaching probes to a particle set, all the particles in the set have the same "code." Thus, a detector would associate the signals for hybridization to the particular attached probes with the "code" for all the particles in the set. However, it is also possible to use two or more "codes" for the same particular probe. Software (or even manual computation) can be used to then associate signals for the two "codes" with the particular probe. In the extreme, each particle has a unique serial number, i.e., its own code. So long as a database tracks which particle codes are modified with a particular probe, data from the detector can be used to associate signals with hybridization to the probe.

### Labeling

**Sample labeling.** A variety of methods can be use to label the sample DNA, e.g., the genomic DNA for analysis. Labels can be introduced by polymerization using nucleotides that include at least some modified nucleotides, e.g., biotin, digoxygenin, fluorescein, or cyanine modified nucleotides. In some aspects, the label is introduced by random-priming and polymerization. Other examples include nick translation (Roche Applied Science, Indianapolis IN; Invitrogen, Carlsbad CA) and chemical labeling (Kreatech ULS, Amsterdam NL). Generally, any labeling method appropriate for labeling genomic DNA samples can be used.

**Shared reference.** In many aspects, the methods evaluate the ratio of the signals between a known reference sample and an unknown experimental sample. For instance, two indirect labels (biotin and fluorescein) are used to allow competitive hybridization of two samples. After hybridization, the sample is divided into two (or more) portion and each portion is evaluated separately. Results from the evaluation can be used to provide the ratio of the two indirect label levels. This approach has the advantage of using the competitive hybridization to normalize any variation between assays: both of the reference and experimental samples are assayed simultaneously in the same vessel mixed with the same particles.

**Parallel Reference.** It is also possible for some aspects to keep the known and unknown samples separate. Competitive hybridization is not used. In this case the reference sample is assayed in one vessel and at least one experimental sample is assayed in another vessel. For example, the vessels can be different wells of a multi-well plate. If multiple experimental samples are used, each can be evaluated in a different vessel, for example a different well of a multi-well plate. The ratio(s) of the experimental sample signals to the reference sample signals for each probe can be obtained in the same way as in the competitive assay.

When this approach is utilized, a single reference sample can be shared between several or many experimental samples. For experiments involving multiple samples per day there can be a savings on reagent cost and labor by avoiding the labeling of multiple duplicate normal samples. Also it is unnecessary to manipulate the sample to obtain different portions for separate analysis. Each sample can be evaluated only once.

**Non-Covalently Attached Labels.** In yet another aspect described herein, the covalent labeling of each sample individually is avoided. For example, unlabeled genomic DNA samples are hybridized to the capture probes immobilized to the encoded particles, wherein said capture probes comprise BAC DNA or oligonucleotide mixtures as described above. Pre-labeled reporter sequences are also hybridized to the probe-sample complexes at sequences adjacent to but not overlapping the sequences of the capture probes. These labeled reporter sequences can be hybridized in the same or in a different hybridization reaction. In this manner the labeled reporter sequences can be manufactured in bulk in a larger-scale environment, lowering the cost per assay compared to individually labeling each sample at the time of the assay.

### Detection

Signals that are indicative of the extent of hybridization can be detected, for each particle, by evaluating signal from one or more detectable labels. Particles are typically evaluated individually. For example, the particles can be passed through a flow cytometer. Exemplary flow cytometers include the Coulter Elite-ESP flow cytometer, or FACScan™ flow cytometer available from Beckman Coulter, Inc. (Fullerton CA) and the MOFLO™ flow cytometer available from Cytomation, Inc., Fort Collins, Colo. In addition to flow cytometry, a centrifuge may be used as the instrument to separate and classify the microparticles. A suitable system is that described in U.S. Pat. No. 5,926,387. In addition to flow cytometry and centrifugation, a free-flow electrophoresis apparatus may be used as the instrument to separate and classify the microparticles. A suitable system is that described in U.S. Pat. No. 4,310,408. The particles may also be placed on a surface and scanned or imaged.

### Exemplary Applications

The methods of genomic evaluation described herein have a variety of applications, including diagnostics and forensics. For example, they can be used to determine the genomic content of an adult, a germ cell, a placental cell, or a fetal cell. The cell can be from any species, but is typically from a diploid species or a species with greater ploidy. For example, the cell can be from a plant (e.g., a crop plant) or animal (e.g., a human or domesticated animal).

With respect to human medical use, the methods can be used to evaluate a sample from a patient, e.g., a sample from a biopsy, a blood sample, an amniotic fluid sample, or a cheek swab. In particular, the patient can be a patient at risk for or having a cancer. The sample can be a sample from a tissue that is near a tumor or from a tumor. For example, the methods can be used to evaluate the genomic content of cells from a carcinoma or sarcoma or from a tumor of the lung, breast, thyroid, lymphoid, gastrointestinal, genito-urinary tract, an adenocarcinomas, e.g., a malignancy of colon cancer, renal-cell carcinoma, prostate cancer and/or testicular tumors, non-small cell carcinoma of the lung, cancer of the small intestine and cancer of the esophagus. Many chromosomal loci that are altered in cancer are known. See e.g. Dutrillaux et al. Cancer Genet. Cytogenet. 49:203-217 (1990), US 5,670,314 (lung carcinomas), US 5,635,351 (gliomas), and US 6,110,673. The method can be used to evaluate the genomic content of a blood cell, e.g., a B or T cell, or a cell from a leukemia or lymphoma.

The methods described herein can also be adapted for other nucleic acids, e.g., DNA other than genomic DNA, mRNA, or other RNAs.

Some aspects of the inventions are further illustrated by the following nonlimiting examples.

### EXAMPLES

### Example 1

In this exemplary ratiometric assay, particles are used to evaluate on multiple samples in parallel with a single label. For example, different samples are places in separate vessels, such as two separate microplate wells. One or more control reagents are used in each vessels to normalize the results between the two.

An exemplary multi-binding assay using a single label readout system is described as follows. A multiplex multi-binding particle assay is performed with two samples I and II, for example. The assay can be extended to additional samples. Each sample is labeled with an indirect label, such as biotin on sample I and dinitrophenol (DNP) on sample II. The specific analytes from both samples compete for bind to the nucleic acid probes on each particle type. After incubation and washing, the particle set is divided into two vessels for separate incubation with two specific secondary labels. In the first vessel fluorescently labeled streptavidin is added and incubated, and, in the second, fluorescently labeled anti-DNP is added and incubated. For the LUMINEX xMAP™ system, a preferred fluorescent label is phycoerythrin. Particles in each of the incubated particles sets with bound fluorescently labeled products I and II are evaluated sequentially by the single-color detection system that associates particle code with phycoerythrin signal.

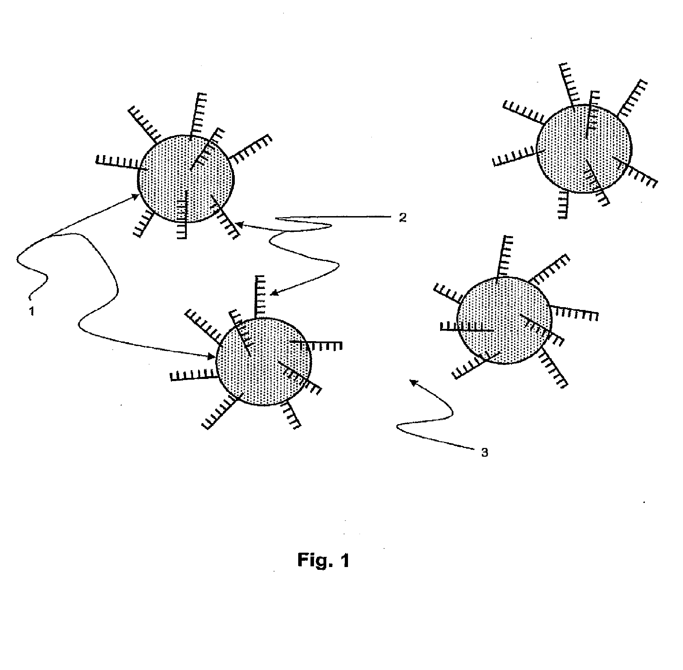

Referring to Fig. 1, assay beads 1 have capture molecules 2 immobilized on their surfaces. In the figure, the capture molecules are shown schematically as a linear nucleic acid sequence for the purpose of this example, but the capture molecules could be any nucleic acid member of a specific binding pair such as cloned DNA or oligonucleotides. The assay beads are suspended in a first liquid assay buffer 3.

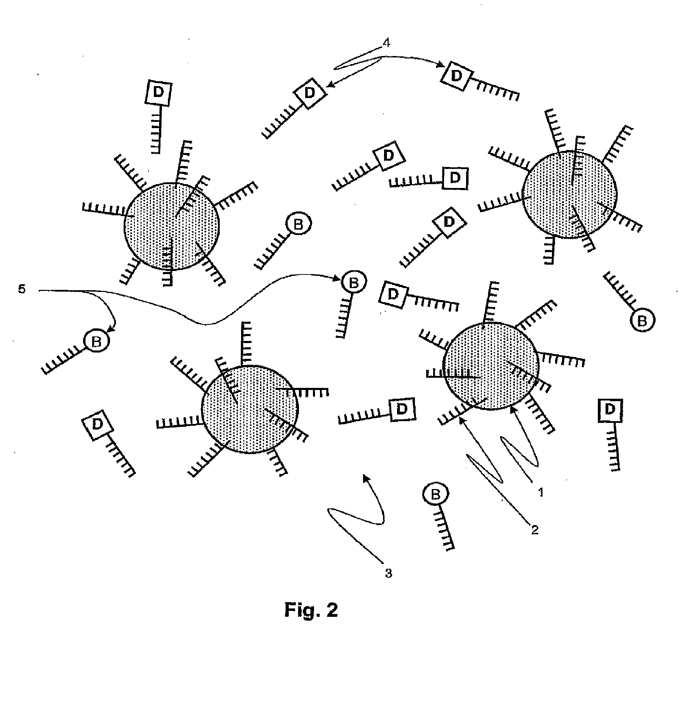

In FIG. 2, two labeled samples are added to the bead suspension of FIG. 1. In this example, the samples are shown schematically as nucleic acid sequences as would be found in gene expression or comparative genomic hybridization assays, but the technique would work with any type of specific binding assay. In the figure, the molecules 5 from a first sample are labeled with a first indirect label B (for biotin in this example), and the molecules 4 from a second sample are labeled with a second indirect label D (for dinitrophenol [DNP] in this example). In this example, there are twice as many "D" labeled molecules 4 as "B" labeled molecules 5. The sample molecules will compete for binding to their immobilized binding pair complements on the beads.

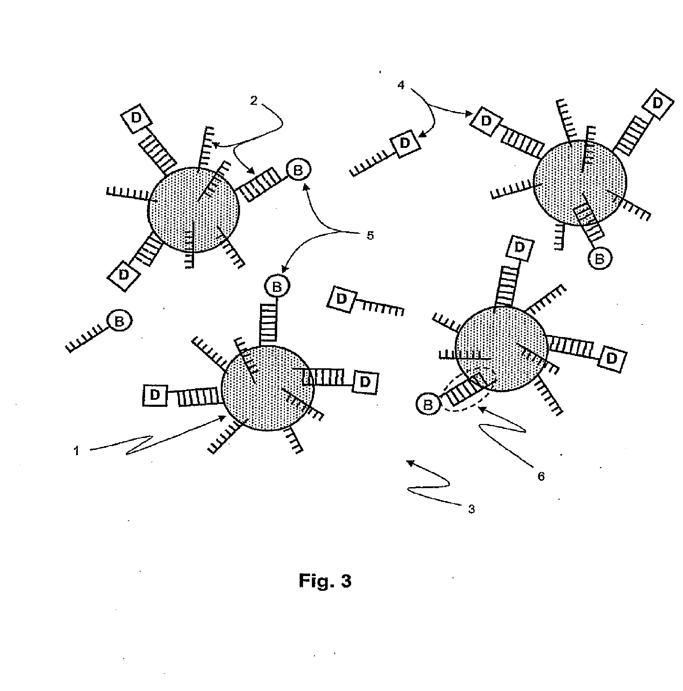

FIG. 3 depicts the beads and indirectly labeled sample molecules from FIG. 1 after incubation and specific binding of sample molecules to capture molecules on the beads. The labeled sample molecules 4 and 5 have competed for binding on capture molecules 2, and have formed bound complexes 6 where binding has occurred. The number of specific binding events on each bead is approximately proportional to the concentration of complementary sample molecules; in this example twice as many of the "D" labeled sample molecules 4 are captured on each bead compared to the "B" labeled sample molecules 5.

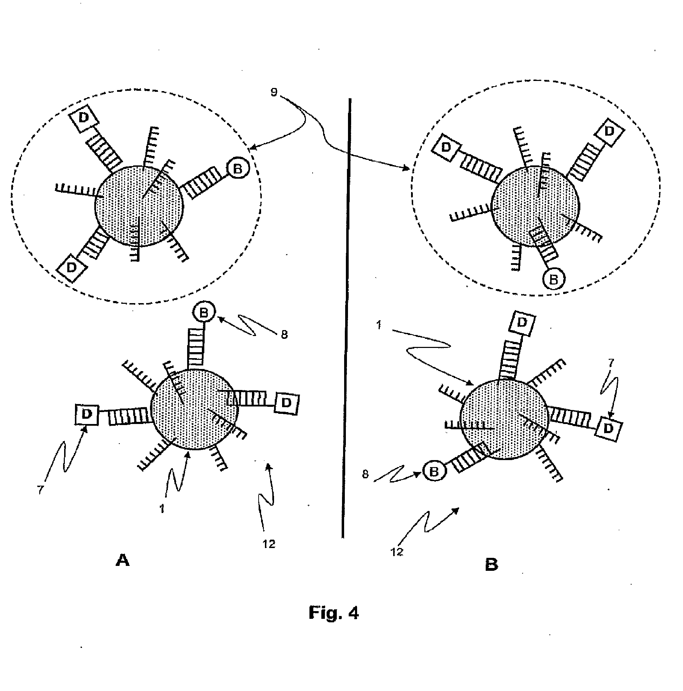

In FIG. 4, the bead complexes 9 with their captured indirectly labeled analytes 7 and 8 have been washed and resuspended in a second buffer 12. Washing removes any sample molecules that have not been specifically bound to the beads. The beads are also shown divided into two aliquots A and B. This is typically done by suspending the beads in the buffer by agitation, then pipetting about half of the volume of bead-buffer suspension into a second vessel, such as a second microplate well. The two aliquots have approximately identical amounts and concentrations of bead complexes 9.

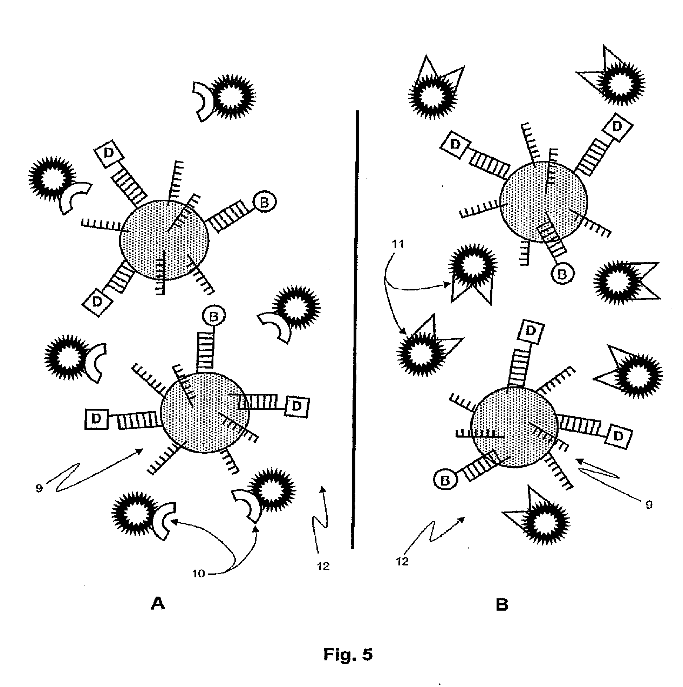

In FIG. 5, different fluorescent label molecule conjugates 10 and 11 complementary to the indirect labels on the sample molecules are added to each aliquot. In aliquot A, streptavidin conjugated to a fluorescent label 10 (such as phycoerythrin or a cyanine or other fluorophore) is added. The fluorescent label is selected to be compatible with the downstream fluorescent bead reading system. In aliquot B, anti DNP conjugated to the same fluorescent label 11 is added. Streptavidin and anti DNP are used in this example as they are complementary to the exemplary biotin and DNP indirect labels, but any high-affinity specific binding pairs can be used.

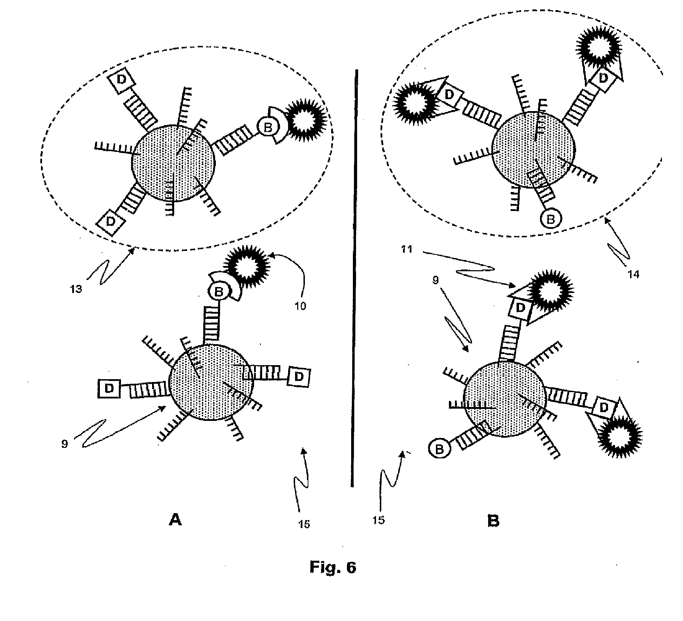

In FIG. 6 the fluorescently labeled bead conjugates 13 and 14 have formed after incubation of the mixtures depicted in Fig. 5, followed by washing and resuspending of the bead complexes in a third buffer 15. Each aliquot contains fluorescently labeled bead complexes, with the population of fluorescent labels on the beads in each aliquot approximately proportional to the concentration of analyte in one of the assayed samples. Each aliquot can now be read individually be a single-label fluorescent bead reading system, such as a LUMINEX xMAP™ system, to produce signals proportional to the fluorescent label density on each bead.

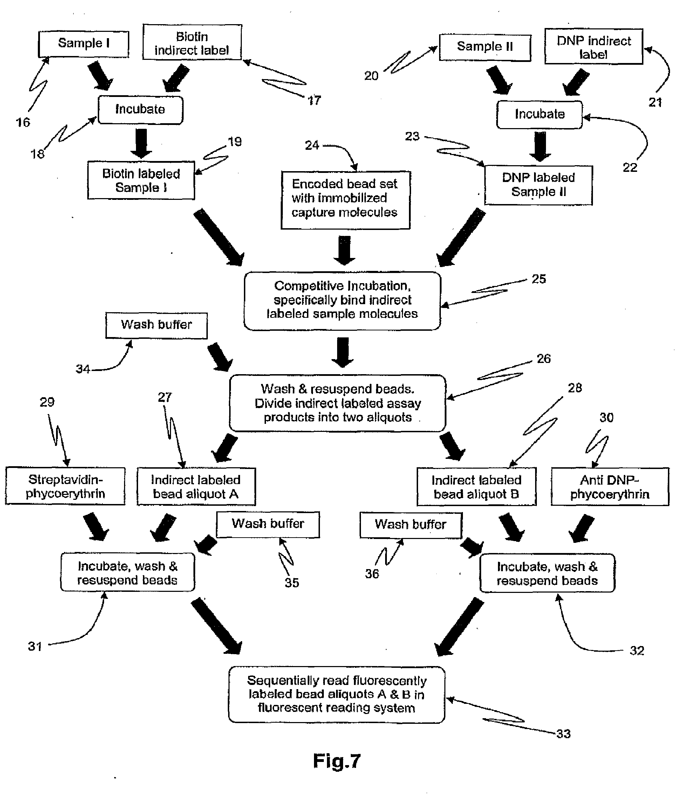

FIG. 7 is a process flow chart of the example assay described in FIGS. 1-6. A first sample 16 is incubated 18 with a first indirect label 17, biotin in this case, to produce a first indirectly labeled sample 19. Separately, a second sample 20 is incubated 22 with a second indirect label (DNP) 21 to produce a second indirectly labeled sample 23. These two samples with indirect labels, 19 and 23, are then incubated in a competitive manner 25 with an encoded bead set 24, where the bead set may be, for example, a set of encoded LUMINEX beads with a different capture molecule immobilized on each bead type (bead "region" in LUMINEX' terminology). After the competitive incubation 25, the beads are washed and resuspended 26 with wash buffer 34, and the resuspended beads are divided into two aliquots 27 and 28.

A fluorescently labeled specific complement to each of the indirect labels is then added separately to each aliquot. In this example, streptavidin-phycoerythrin 29 is incubated 31 to the first indirect bead aliquot 27, where the streptavidin specifically binds to the biotin indirect labels on the bead complexes. Separately, anti DNP-phycoerythrin 30 is incubated 32 with the second bead aliquot 28, where the anti DNP specifically binds to the DNP indirect labels on the bead complexes. Next, and still keeping the two aliquots separate, excess fluorescent conjugate is washed away 31 and 32 with wash buffer 35 and 36, Finally, each fluorescently labeled bead aliquot is read 33 separately, using a LUMINEX xMAP 100™ or xMAP 200™ instrument in this example.

### Example 2

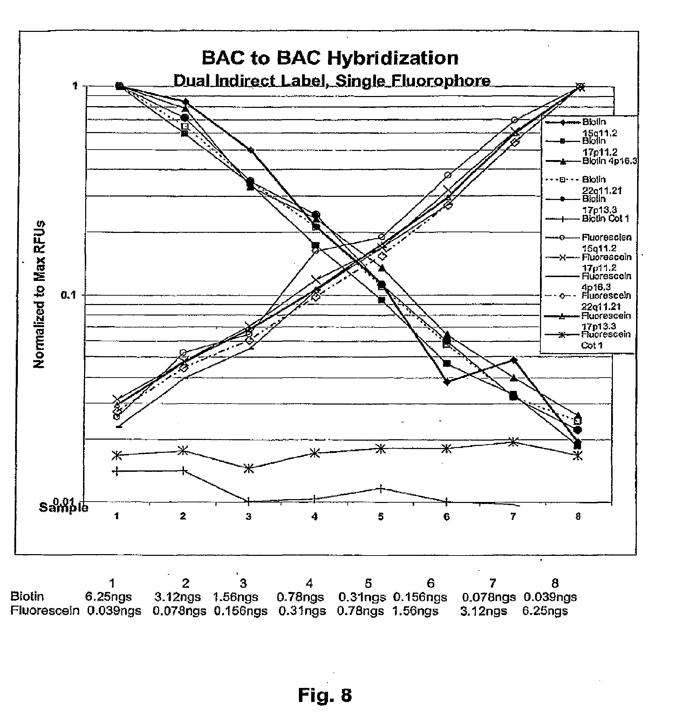

FIG. 8 through 10 depict results of exemplary reference assays. FIG. 8 demonstrates the specificity and sensitivity of five-plex BAC-CGH assays run on the LUMINEX® platform with biotin and fluorescein as the indirect labels. First, fragmented DNA from each of five BACs was immobilized on five sets of LUMINEX beads, by initially modifying the carboxylated bead surface to an amino surface, and then using 1-Ethyl-3-[3-dimethylaminopropyl]carbodiimide hydrochloride (EDC or EDAC chemistry) to immobilize the 5' ends of the BAC fragments onto the beads. The six bead sets, each with a different LUMINEX bead ID or "region" were then pooled into a multiplex bead set. These steps are detailed as follows:
**BAC probes, standards, and samples.** Bacteria artificial chromosome (BAC) DNA was used to make immobilized capture probes on encoded beads. Five different human BACs, all supplied by the Health Research Division, Roswell Park Cancer Institute (Buffalo, NY), were used as immobilized capture probes in the following experiments: RP11-289D12 (15q11.2); RP11-524F11 (17p11.2); RP11-1398P2 (4p16.3); RP11-476-C20 (22q11.21); RP5-59D14 (17p13.3). These BACs are listed by Roswell Park clone # with the chromosomal locus in parentheses.

These BACs were used as standards in the experiments. A pool of the five BACs was used to make enzymatically labeled positive samples using Klenow random-primer labeling with biotin-labeled and unlabeled nucleotides. Additionally, Cot-1 DNA (Invitrogen 15279-011, Carlsbad CA) was immobilized to encoded beads as a negative control.

BAC probe preparation and immobilization onto beads. BAC DNA was immobilized on LUMINEX xMAP™ beads from Luminex (Austin TX). xMAP™ beads are made of polystyrene functionalized with carboxyl groups on the surface. These beads are offered in both para-magnetic and non-magnetic versions. The paramagnetic beads facilitate separating the beads from solution for wash steps, but the same protocol works for non-magnetic beads where washes are performed on filter columns or plates (e.g. HTS 0.45 µm, catalog MSHVN4510, Millipore, Billerica MA).

The BAC and control probe immobilization protocol onto magnetic LUMINEX beads was as follows.
Amino conversion of carboxylate beads by reaction with 4,9-Dioxa-1,12-dodecandiamine and Ethyl-3-[3-dimethylaminopropyl]carbodiimide Hydrochloride (EDC).
1. Dispensed 250 µl (2.5 × 10⁶ beads) from LUMINEX standard shipping/storage vial of six bead regions (24, 46, 47, 56, 68 and 79) into six separate 1.5 ml Eppendorf tubes using siliconized 200 µl pipette tips.
2. Added 750 µl of 0.1M 2-(N-morpholine)-ethane sulphonic acid (MES) 0.15M NaCl pH 6.0 to each tube. Vortexed and sonicated each tube.
3. Pelleted magnetic beads in each tube by contacting a samarium-cobalt magnet disc magnet to the side of the vial for 10 minutes. Carefully removed 900 µl of liquid while avoiding disturbance of the magnetic beads.
4. Adjusted volume to 80 µl total with MES in each tube. Added 10 µl 4,9-Dioxa-1,12-dodecandiamine (at 50 µl/ml MES) and 10 µl EDC (50 mg/ml in MES) to each tube.
5. Vortexed and sonicated each tube to re-suspend beads.
6. Incubated for 120 minutes at room temperature on tube rotator.
7. Added 900 µl of PBS to beads, vortexed and sonicated.
8. Pelletized magnetic beads using magnet for 5 minutes. Removed 1,000 µl liquid with transfer pipette, added 1,000 µl Phosphate buffered saline (PBS). Vortexed and sonicated each tube to re-suspend beads.
9. Washed each tube of beads 3 × by pelletizing with magnet, drawing off liquid, adding wash buffer (1xPBS 0.01% BSA 0.01% Tween 20), sonicating and vortexing. Final buffer addition was 250 µl 1xPBS 0.01% BSA for storage.

EDC coupling of BAC and control DNA onto beads:
1. Aliquotted 0.5×10⁶ amino beads (50 µl) of each of the six bead regions into 1.5 ml Eppendorf tubes, vortexed and sonicated.
2. Added 5.0 µl BAC DNA or Cot-1 DNA(fragmented by sonication)(nominal concentration 50 ng/µl) to respective tubes, vortexed briefly. Added 5 µl freshly dissolved EDC (10 mg/ml in dH2O). Vortexed immediately, then incubated 30 minutes at room temperature in the dark. Repeated EDC addition, vortexing and 30 minute incubation. Added 700 µl 0.2% Tween 20 in dH₂O vortexed.
3. Pelletized magnetic beads using magnet for 5 minutes. Removed supernatant. Re-suspended beads in 900 µl 0.05% Tween 20 in dH₂O, vortexed and sonicated. Heated at 100C for 5 minutes to denature double stranded DNA coupled to the surface. Repeated this step one more time.

Preparation of multiplex bead mix
1. Vortexed & sonicated each of the 6 tubes of beads with immobilized BAC and Cot-1 control probes. Pipetted aliquot from each bead tube into a new tube with an estimated bead yield of 2,500 beads/µl.
2. Pelletized the multiplex bead mix beads with magnet for 10 minutes. Added 200 µl warmed AMBION® Slide Hybridization Buffer #2 (Ambion, Austin TX). Bead mix used in aliquots of 10 µl, enough for 20 hybridizations with approximately 2,500 beads of each region in each aliquot.

For a demonstration assay, sample DNA was prepared by pooling the same BAC DNA fragments that were used to prepare the multiplex bead. The BAC DNA, being double-stranded, will hybridize specifically with themselves after denaturing, allowing standard curves to be generated with samples of known concentration.
Dilutions of at each of eight concentrations, in two-fold dilutions down from 6.25 ng/ml, were used as standards. Each of these DNA dilutions was divided into two aliquots, and each of the two aliquots labeled with either fluorescein or biotin to create indirectly-labeled samples.

Labeling was done using a standard BioPrime™ BAC planar microarray random primer labeling kit (Invitrogen, Carlsbad CA) substituting biotin-labeled and fluorescein-labeled nucleotides for the standard cyanine dye labeled nucleotides.
Details are as follows:
1. Pooled aliquots of each of the five BACs into a single tube, vortexed.
   Added 2 µg of each pool to two microfuge tubes. Adjusted the volume to 50 µl with deionized H₂O.
2. Added 40 µl of 2.5X Random Primer Mix from the kit to each of the two labeling reactions. Vortexed for 2 seconds, centrifuged for 10 seconds.
3. Denatured the DNA on a heat block for 5 minutes at 100°C. Snap-cooled in ice slurry for 5 minutes, centrifuged for 10 seconds.
4. Made biotin master mix (enough for six reactions)
   16 µl Spectral Labeling Buffer (unlabeled dNTP nucleotide mix in EDTA and tris buffer, Spectral Genomics, Houston TX)
   10 µl biotin dCTP
   6.5 µl Klenow (Invitrogen, Carlsbad CA)
   32.5 µl total
5. Added 10 µl of master mix to each tube, centrifuged for 10 sec and incubated each at 37°C for 2 hours.
6. Removed 3 µl from each reaction and ran electrophoresis on an agarose gel to confirm that labeled product was approximately 100 bp in length.
7. Added 8 µl EDTA in dH₂O, pH 8.0, incubated at 70°C for 10 minutes.

Placed labeled samples on ice.
8. Added 90 µl Spectral Genomics Hybridization Buffer 1 (Human Cot-1 DNA and sheared salmon testis), added 38.8 µl 5M NaCl, and vortexed. Added 260 µl isopropanol, vortexed and centrifuged.
9. Incubated at room temperature for 20 minutes. Centrifuged at full speed for 20 minutes. Stored at 4°C until needed.
10. Prior to use, the labeled BACs were warmed to room temperature and centrifuged for 10 minutes.
11. Removed supernatant and added 1,000 µl of 70% ethanol 30% d.

Centrifuged for additional 3 minutes and removed all liquid, leaving pellet.
12. Air dried for 10 minutes, or until visibly dry.
13. Added 20 µl deionized H₂O to pellet, incubated 10 minutes at room temperature. Estimated concentration of labeled DNA: 2 µg/20 µl (100 ng/µl).

The above protocol was repeated with fluorescein nucleotides replacing the biotin nucleotides in a second fluorescein master mix at Step 4. The products of these two operations were: a biotin labeled BAC pool and a fluorescein labeled BAC pool. These comprised labeled pools of the same BACs used as probes, which would be perfect-match positive hybridization complements to the immobilized probes. The same procedures can be used for sample of nucleic acid prepared from a cell, e.g., genomic DNA from a patient or other subject.

Eight pairs of the indirectly labeled samples were then each hybridized to the LUMINEX multiplexed bead set, the eight pairs being made up of increasing concentrations of fluorescein-labeled sample mixed with decreasing concentrations of biotin-labeled sample, per the notations along the horizontal axis of the plot in Fig. 8. As detailed below, the hybridization was performed at 50°C for two hours, and was followed by three stringency washes, also at 50°C. The stringency wash buffers were, in order, 2X SSC + 50% deionized formamide; 2X SSC + 0.1% Igepal; and 0.2X SSC.

### Hybridization & Detection

1. Made four dilution series of labeled samples in 1.5 ml microcentrifuge tubes. Mixed reciprocal pairs of same-label dilutions to form the following 8 samples:

| Sample # | 5 BAC Pool, fluorescein | | 5 BAC Pool, biotin |
|---|---|---|---|
| 1 | 0.039 ng | + | 6.25 ng |
| 2 | 0.078 ng | + | 3.12 ng |
| 3 | 0.156 ng | + | 1.56 ng |
| 4 | 0.31 ng | + | 0.78 ng |
| 5 | 0.78 ng | + | 0.31 ng |
| 6 | 1.56 ng | + | 0.156 ng |
| 7 | 3.12 ng | + | 0.078 ng |
| 8 | 6.25 ng | + | 0.039 ng |

2. Dried down volumes of labeled samples in a SpeedVac™ (Kendro Laboratory Products, ThermoElectron Corp, Asheville NC). Added 10 µl of warmed multiplex bead mix as prepared above. Mixed with pipette and denatured on a block heater at 98C for 2 minutes
3. Hybridized by incubating microtubes inside a 50 ml centrifuge tube covered with aluminum foil, placed on a rotating rack in a 50C oven for 2 hours.
4. After hybridization, added 1 ml of 0.2x SSC to each sample tube and vortexed.
5. Transferred the contents of each tube to 2 wells of a 96-well PCR plate, splitting each hybridized bead set into a pair of 500 µl aliquots.
6. Pelletized the beads in the plate wells with a magnet for 10 minutes, and 500 µl supernatant was carefully removed to avoid bead loss.
7. Added 100 µl of 8 µg/ml PJ31S streptavidin-phycoerythrin (Prozyme, San Leandro CA) to one well of each pair, and added 100 µl 8 µg/ml antifluorescein-phycoerythrin (Invitrogen, A-21250) to the other well of each pair. Then, incubated the plate while agitating at room temperature on an NCS™ Shaker Incubator (PerkinElmer, Boston MA) at 950 RPM for 30 minutes, and followed by washing the beads with 1x PBS + 0.01% Tween 20 buffer..
8. Pelletized the beads in each plate well with a magnet for 10 minutes, and 100 µl supernatant was carefully removed to avoid bead loss. Added 100 µl Tris-NaCl-Tween 20 buffer, mixed by re-pipetting to re-suspend beads.
9. Read the samples on a LUMINEX 100™ analyzer, recording the median fluorescence intensity for each bead region in each well.

As detailed above in steps (7-9), after the two-sample competitive hybridization assays using the indirect labels were completed, the multiplex assayed bead sets were divided again. One aliquot was secondarily labeled with 4 µg/ml anti-fluorescein-phycoerythrin and the other with 4 µg/ml streptavidin-phycoerythrin. Each of these secondarily labeled bead sets was then read separately on a LUMINEX xMAP 200™ system.

The results are shown in Fig. 8. The signal from each BAC is shown as a separate data trace, and the fluorescein-labeled samples are shown separately from the biotin-labeled ones. Each shows a linear response of concentration to signal over an approximately 160:1 range of concentrations. The largest fluorescein-labeled signals do not appear to interfere with the lowest biotin-labeled signals, and vice-versa. Also, signals from the beads with the COT-1 immobilized on them are lower than the lowest signals from the specific binding events. Such signals were less than 2% of full scale under all conditions. This demonstrates that the two indirect labels do not exhibit substantial interference or cross-reactivity in these concentration ranges.

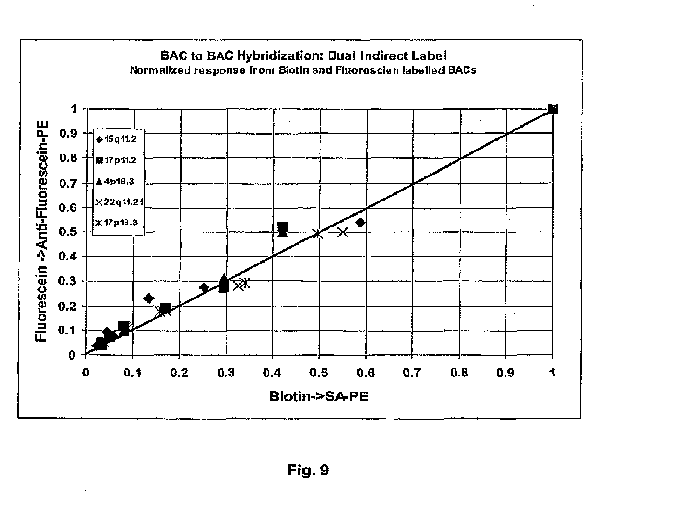

Fig. 9 shows the data from Fig. 8 formatted in a different manner. This is a "same-same" scatter plot, of the type often used in differential gene expression microarray analysis. The scales on both axes are normalized concentrations of each of the biotin- and fluorescein-labeled BAC samples. In an idealized or perfect assay, each data point would be on the diagonal line: the signal from each competitively-assayed indirectly-labeled sample would be the same, since the concentrations are the same. The actual data is closely grouped near the diagonal line, with no more than a 1.5-fold deviation from the ideal and typically less. Standard CGH (or gene expression, as well) assays commonly use 2:1 differential signal as the threshold indicating biological significance.

### Example 3

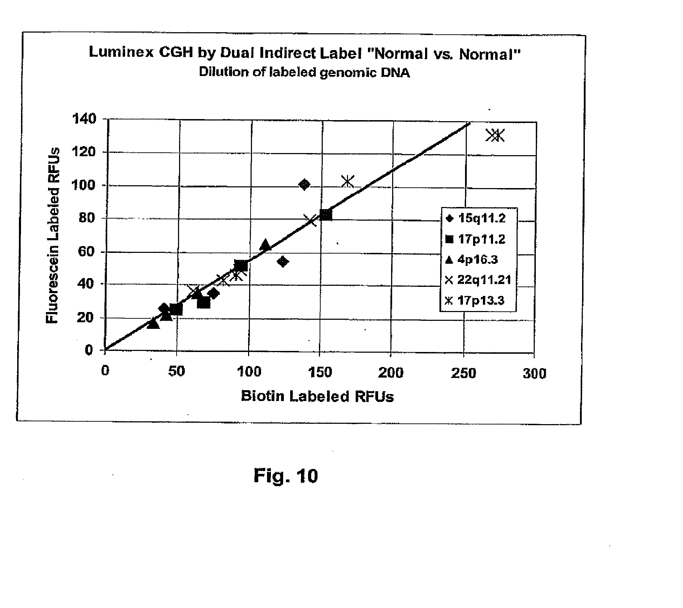

Fig. 10 shows the results of a 5-plex CGH assay, using the same multiplex bead set described above, on indirectly labeled genomic DNA samples. A genomic DNA sample (Human Genomic DNA: Male; Promega Corporation, Madison WI) was divided into two aliquots. One aliquot was indirectly labeled with fluorescein and the other with biotin using the BioPrime™ kit referenced above to create two indirectly labeled samples. The two indirectly labeled samples were then mixed, and assayed competitively together against the multiplex bead set. After the competitive assay using the indirect labels the bead set was divided, and one aliquot was secondarily labeled with anti-fluorescein-phycoerythrin and the other with streptavidin-phycoerythrin. Each of these secondarily labeled bead sets was then read on a LUMINEX xMAP 200™ system. The resulting data, again, is formatted as a "same-same" scatter plot in Fig 10. Like the deliberately constructed dilution series shown in Fig. 9, the data from the divided sample of genomic DNA is closely congruent to the diagonal line on the plot.

### Example 4

In a exemplary assay that illustrates another aspect of the invention, synthetic 70-mer amine-modified oligonucleotide probes (Operon Biotechnologies, Huntsville AL) were immobilized to the Luminex multiplex beads and a single-color assay was performed where the reference and test samples were not in the same well. This experiment utilized 17 different probes, four of which were pooled or combined sets of five oligonucleotides representing loci on the X and Y chromosomes as detailed in the table below. With this multiplex probe set a demonstration assay that differentiates male and female DNA was performed.

**Table 1.**

| **Bea d ID** | **Gene(s)** | **Chromosome location** | **Oligonucleotide Probe Sequences** |
|---|---|---|---|
| **52** | **ZFY USP9Y EIF1AY CYorf15B PCDH11Y** | | |
| | | **5Y combine d** | |
| | | | |
| | | | |
| | | | |
| **43** | **Q96MI2_ HUM UTY TTTY14 Y9 Y10** | **5Y combine d** | |
| | | | |
| | | | |
| | | | |
| | | | |
| | | | |
| | | | |
| | **PHF6_HU M GPC3 RNF127 DUSP9 NP_87241 3.1** | | |
| **63** | | **5X combine d** | |
| | | | |
| | | | |
| | | | |
| | **CDX4 MAGEH1 ALAS2 IL13RA1 XP_37225 7.1** | **5X combine d** | |
| **56** | | | |
| | | | |
| | | | |
| **9** | **MCL1** | **1q21.2** | |
| **40** | **CASP3** | **4q34** | |
| **62** | **BAK1** | **6p21.31** | |
| **44** | **TNF** | **6p21.33** | |
| **10 0** | **CYC1** | **8q24.3** | |
| **2** | **BAG1** | **9p12** | |
| **36** | **TRAF2** | **9q34** | |
| **85** | **BNIP3** | **10q26.3** | |
| **1** | **TNFRSF1 A** | **12p13.2** | |
| **76** | **TNFAIP2** | **14q13.32** | |
| **29** | **AKT1** | **14q33.3** | |
| **10** | **BAX** | **19q13.3** | |
| **6** | **NUP62** | **19q13.33** | |

The protocol for coupling the amine-modified oligonucleotide probes to the encoded Luminex xMap microspheres or beads to make the multiplex oligo bead mix was as follows.
1. Bring a fresh aliquot of -20°C, desiccated EDC (1-Ethyl-3-[3-dimethylaminopropyl] carbodiimide Hydrochloride) powder (Pierce Biotechnology, Rockford IL) to room temperature.
2. Resuspend the 70 mer C-6 amine-modified oligonucleotide to 100 µM (100 picomole/µL) in dH₂O.
3. Resuspend the stock xMAP microspheres by light vortex and sonication for approximately 20 seconds.
4. Transfer 1,250,000 beads of the stock microspheres (100 µL) to a 1.5 ml Seal-Rite microfuge tube (USA Scientific, Ocala FL).
5. Pellet the stock microspheres by microcentrifugation at ≥ 8000 x g for 3-5 minutes (enough time for solid pellet formation).
6. Remove the supernatant and resuspend the pelleted microspheres in 50 µL of 0.1 M MES buffer, pH 4.5 by sonication for approximately 20 seconds (enough time so that no pellet or smearing on the tube is visible).
7. Dilute the 100 µM amine oligonucleotide 1:4 (1 µL into 3 µL dH₂O) for a final concentration of 25 µM (25 pmoles/µL).
8. Add 1.0 µL of the capture oligo (25 pmoles) and mix by sonication for approximately 20 seconds.
9. Prepare a fresh solution of 10 mg/mL EDC in dH₂O. (Note: Return the EDC powder to desiccant to re-use for the second EDC addition).
10. One by one for each reaction, add 2.5 µL of fresh 10 mg/mL EDC to the microspheres (25 µg or ≈ [0.47 µg/µL] _{final}) and mix by sonication for approximately 20 seconds.
11. Incubate for 30 minutes at room temperature in the dark, sonicating at the 15 minute mark (to reduce settling of microspheres).
12. Prepare a second fresh solution of 10 mg/mL EDC in dH₂O. (Note: The aliquot of EDC powder should now be discarded. We recommend using a fresh aliquot of EDC powder for each coupling episode).
13. One by one for each reaction, add 2.5 µL of fresh 10 mg/mL EDC to the microspheres and mix by sonication for approximately 20 seconds.
14. Incubate for 30 minutes at room temperature in the dark, sonicating at the 15 minute mark.
15. Add 1.0 mL of 0.02% Tween-20 to the coupled microspheres and mix by vortex and sonication.
16. Pellet the coupled microspheres by microcentrifugation at ≥ 8000 x g for 3-5 minutes.
17. Remove the supernatant and resuspend the coupled microspheres in 1.0 mL of 0.1% SDS buffer by vortex and sonication.
18. Pellet the coupled microspheres by microcentrifugation at ≥ 8000 x g for 6-10 minutes.
19. Remove the supernatant and resuspend the coupled microspheres in 100 µL of 10 mM Tris, 1 mM EDTA, pH 8.0 by vortex and sonication for approximately 20 seconds.

Assuming 100% recovery, the resulting oligo-coupled bead concentration is ∼12,500 microspheres/µL.

Standard normal genomic DNA, pooled from a number of nominally normal individuals of the same sex (Promega, Madison WI) was used as the test samples in this assay. Male DNA was used as the test sample and female DNA for the reference. Labeling 2 µg of each sample was performed with the Invitrogen BioPrime kit and Spectral Genomics reagents as in Example 4 above, except that biotin nucleotides were substituted for the cyanine nucleotides in the kit to yield biotin-labeled samples.

The multiplex demonstration assay was performed on the Luminex xMAP beads with both samples in duplicate, utilizing four wells in a 96-well microplate per the protocol below.
1. For each sample, add 1.0 µg of biotin labeled human genomic DNA to a well of a Matrix (Hudson NH) polypropylene 96 well V-bottom polypropylene microplate.
2. Add 10.0 µg of Cot 1 DNA & 10.0 µg of salmon sperm (Invitrogen, Carlsbad CA) to each well of V-bottom plate to be hybridized. Include a negative control well(s) with no Biotin DNA, but containing nucleic acid blockers (Cot 1 DNA & Salmon Sperm). This will be used to determine the bead blank(s).
3. Dry down the plate(s) in a SpeedVac for 30-120 minutes or until bottom of wells are clear.
4. Hand warm Spectral Genomics Hyb II Buffer to dissolve precipitates and dilute by adding ¾ of the buffer to ¼ of sterile distilled H₂O in a 1.5 mL tube.
5. Add an appropriate amount of the multiplex oligo bead mix into the diluted

Spectral Genomics Hyb II Buffer so that the result is -300 of each bead code/well (enough for 7.5 µL/well). Mix by pipetting up and down.
6. Dispense 7.5 µL of the multiplex oligo bead mix in hybridization buffer to each well of the V-bottom plate(s) to be hybridized. Mix by pipetting up and down.
7. Cap wells with Matrix single strip plate caps and seal plate with a Bio-Rad (Hercules CA) plate cover.
8. Denature samples in the plate(s) on a 96 well thermal cycler @ 100°C for 2 minutes, then cool to 50°C for 2 minutes with no heated lid.
9. Place plate(s) in a PerkinElmer NCS microplate incubator, and incubate overnight @ 50° and 1150 rpm shaking.
10. After hybridization, add 100 µL of 2X SSC, 50% formamide (Spectral Genomics Stringency Wash, heated to 50°C prior to use) to each reaction and incubate plate(s) in a PerkinElmer NCS Plate Incubator for 20 minutes @ 50° and 1150 rpm shaking.
11. Wet all wells of a Millipore 0.45 µm HT filter plate(s) with 30 µL 0.2X SSC for uniform vacuum filtration.
12. Transfer volumes from the Marix V-bottom plate(s) to the Millipore filter plate(s) for washing.
13. Gently apply vacuum using Millipore vacuum manifold to remove liquid and pat dry the bottom of the filter plate with a paper towel.
14. Add 100 µL of 2X SSC, 0.1% Igepal (Spectral Genomics Stringency Wash, heated to 50°C prior to use) to each reaction, cover top (loosely) with aluminum foil and incubate plate(s) in a PerkinElmer NCS microplate incubator for 20 minutes @ 50°C and 1150 rpm shaking.
15. Gently apply vacuum using vacuum manifold to remove liquid and pat dry the bottom of the filter plate with a paper towel.
16. Add 100 µL of 0.2X SSC (Spectral Genomics Stringency Wash, heated to 50°C prior to use) to each reaction, cover top (loosely) with aluminum foil and incubate plate(s) in a PerkinElmer NCS Mixer for 10 minutes @ 50°C and 1150 rpm shaking.
17. Gently apply vacuum using vacuum manifold to remove liquid and pat dry the bottom of the filter plate with a paper towel.
18. Add 100 µL 1X PBS, 0.1% BSA, 0.05% Tween with 4.0 µg/mL of PhycoLink^{®} Streptavidin-R-PE (Prozyme Lot PJ13S) to each reaction. Cover top (loosely) with aluminum foil and incubate plate(s) in a PerkinElmer NCS Mixer for 30 minutes @ 25°C and 1050 rpm shaking.
19. Gently apply vacuum using vacuum manifold to remove liquid and pat dry the bottom of the filter plate with a paper towel.
20. Add 100 µL 1X PBS, 0.01% Tween 20 to each reaction and apply vacuum to filter plate, patting dry.
21. Add 100 µL 1X PBS, 0.01% Tween 20 to each reaction. Shake filter plate(s) in a PerkinElmer NCS microplate incubator for at least 1 minute at 1050 rpm.
22. Read samples using the Luminex 200 instrument, using median the median fluorescence signal of for each bead region and a minimum bead count setting of 50.

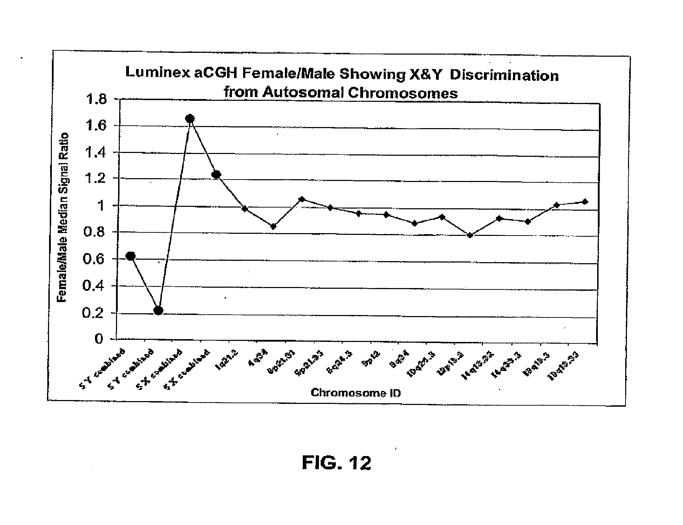

The results of the demonstration assay are shown in FIG. 12, where the ratio of the female samples' signals to the male samples' signals are on the vertical axis and the oligonucleotide probe identities are on the horizontal axis. The signals for the two duplicates of each sample were averaged. The X and Y sex-specific chromosome probes produced signal ratios in excess of 1.2 above or below the unity line, whereas all of the non-sex-specific probes produced ratios of less than 1.2 above and below.

### Example 5

Multiplex differential gene expression is commonly performed on printed microarrays using a two-fluorophore assay. In this type of assay two RNA samples, a reference and a sample to be tested, are enzymatically converted to cDNA by reverse transcriptase in the presence of labeled nucleotides. The cDNA products thus have a fraction of their nucleotides fluorescently labeled whereby they can be detected optically. Each sample is labeled separately with two different fluorophores, typically cyanine 3 and cyanine 5, and the labeled samples are pooled and competitively hybridized to a microarray. The ratio of the two dyes at each element of the microarray, detected on a fluorescence scanning instrument, is thus indicative of the relative concentration of each assayed RNA sequence in its respective sample.

This type of assay was performed on paramagnetic Luminex xMAP™ beads with a single fluorophore readout according to an aspect of the present technology. Oligonucleotide ("oligo") probes representing 38 genes were obtained from Operon (Huntsville AL). These oligo probes were of 70-mer length with an amine group at the terminal 5' end. Each oligo sequence was immobilized onto a set of xMAP beads with a particular xMAP bead ID code or region, using the common EDAC coupling chemistry. The 38 genes represented were NRP2, CARD 14, IGFALS, TSSC3, PSEN2, IGFBP4, TP53BP2, GAPD, PRODH, TNFRSF7, RAB6KIFL, ILF1, BCL2L2, DNASE1L3, PPP1R15A, PIG11, HSPD1, CDH1, IGFBP5, IRF1, TNFRSF10C, TNFRSF17, LTA, DFFB, IL16, PTPN13, IL3, TNFSF18, CCNG1, CCND1, TUCAN, RIPK3, CASP6, IL2, TNFAIP2, IL24, K-ALPHA-1, and TRIP, along with a negative control. The 39 bead types were then pooled into a multiplex bead set, and aliquots from this set were placed into the wells of a 96-well microplate to perform a gene expression assay as described below.

For the purpose of demonstrating the technology, a single reference RNA sample was used (Universal Human Reference RNA, Stratagene, La Jolla CA). The single sample was split into two, and the expected result of a differential assay is thus a ratio of 1:1 for each gene. This is a common evaluation performed on gene expression platforms. According to an aspect of the present technology, one aliquot of RNA was labeled with biotin and the other with fluorescein as indirect labels. These two indirectly labeled samples were then pooled and mixed with the multiplex bead sets prepared previously and allowed to hybridize simultaneously.

After hybridization the assayed bead set was divided into two aliquots. Streptavidin-phycoerythrin reporter reagent (specific to the biotin-labeled sample) was added to an incubated with one aliquot, and anti-fluorescein-phycoerythrin (specific to the fluorescein-labeled sample) was added to and incubated with the other. Phycoerythrin is the preferred reporter fluorophore in the xMAP™ instrument. The paramagnetic bead aliquots were then pulled to the microplate well walls by a plate magnet, the liquid reagent withdrawn by a pipette, and the labeled beads were resuspended in a wash buffer. The two aliquots of beads were then read sequentially on a Luminex xMAP 200™ instrument.

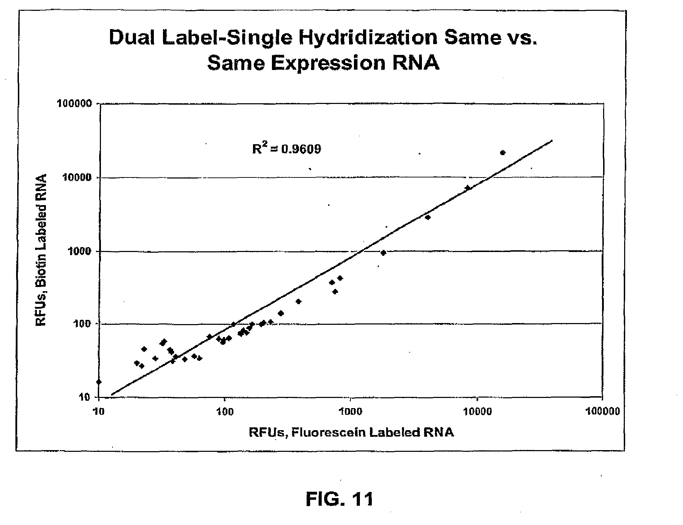

The resulting signal data were plotted on a scatter plot, as shown in FIG. 11. If all of the data were positioned at the identity line, this would correspond to a ratio of 1:1 and a correlation value R² of 1.0. In this demonstration assay, the correlation factor was 0.96. This value is an improvement over values produced by typical printed microarrays using 2-dye detection, which typically produce R² values between 0.92 and 0.96.

## Claims

1. A method of evaluating genomic DNA, the method comprising:
providing a plurality of DNA samples, wherein at least one sample of the plurality of samples is a reference sample, with a known number of copies for each interrogated genomic locus, and at least one sample is a genomic DNA sample;
providing a particle mixture, the mixture comprising particles from different particle sets, wherein each particle set contains numerous encoded particles, wherein each particle within a particle set comprises i) the same code, and ii) a nucleic acid hybridization probe for a particular genomic locus, wherein each different particle set is specific for a different genomic locus, such that the mixture collectively includes probes for a plurality of different genomic loci and wherein the nucleic acid hybridisation probe of each particle comprises BAC nucleic acid, wherein the hybridization probe of each particle is produced by sonicating or fragmenting the BAC DNA;
labelling the reference sample with a first indirect label and labelling the genomic DNA sample and other samples of the plurality of samples with a second indirect label;
contacting each of the labelled samples to a portion of the particle mixture under hybridization conditions; and
evaluating hybridization of each of the samples to particles in the respective portion of the mixture by monitoring the first indirect label or the second indirect label, or both, wherein signals from monitoring the reference sample are compared to signals from other samples to determine the number of copies of each interrogated genomic locus for the samples.

2. The method of claim 1, wherein more than one DNA sample is provided and each of the DNA samples is evaluated.

3. The method of claim 1, wherein the nucleic acid hybridization probe comprises a collection of oligonucleotides specific for a particular chromosomal locus.

4. The method of claim 2, wherein the reference sample comprises a known number of copies for each interrogated genomic locus, and the method comprises comparing signals from monitoring the labelled reference sample to signals from other samples to determine the number of copies of each interrogated genomic locus for the samples.

5. The method of claim 1, wherein chromosomal amplification is detectable.

6. The method of claim 1, wherein a heterozygous deletion of a chromosome is detectable.

7. The method of claim 1, wherein a homozygous deletion of a chromosome is detectable.

8. The method of claim 2, wherein the code is provided by holographic encoding or by a fluorescence property.

9. The method of claim 1, in which the DNA is attached to the particle using a crosslinker.

10. The method of claim 1, in which each sample is contacted to the particle mixture in a separate vessel.

## Patentansprüche

1. Verfahren zur Evaluierung genomischer DNA, wobei das Verfahren umfasst:
Bereitstellen mehrerer DNA-Proben, wobei zumindest eine Probe der mehreren Proben eine Referenzprobe ist, mit einer bekannten Anzahl von Kopien für jeden abgefragten genomischen Lokus, und zumindest eine Probe eine Probe genomischer DNA ist;
Bereitstellen eines Partikelgemischs, wobei das Gemisch Partikel aus unterschiedlichen Partikelsets umfasst, wobei jedes Partikelset zahlreiche codierte Partikel enthält, wobei jeder Partikel innerhalb eines Partikelsets umfasst i) den gleichen Code, und ii) eine Nukleinsäure-Hybridisierungssonde für einen bestimmten genomischen Lokus, wobei jedes unterschiedliche Partikelset für einen anderen genomischen Lokus spezifisch ist, so dass das Gemisch kollektiv Sonden für mehrere unterschiedliche genomische Lozi beinhaltet, und wobei die Nukleinsäure-Hybridisierungssonde jedes Partikels BAC-Nukleinsäure umfasst, wobei die Hybridisierungssonde jedes Partikels durch Ultraschallbehandeln oder Fragmentieren der BAC-DNA produziert wird;
Markieren der Referenzprobe mit einer ersten indirekten Markierung und Markieren der Probe genomischer DNA und anderer Proben von den mehreren Proben mit einer zweiten indirekten Markierung;
In-Kontakt-Bringen jeder der markierten Proben mit einem Teil des Partikelgemischs unter Hybridisierungsbedingungen; und
Evaluieren der Hybridisierung jeder der Proben an Partikel im jeweiligen Teil des Gemischs durch Überwachen der ersten indirekten Markierung oder der zweiten indirekten Markierung, oder beider, wobei Signale aus der Überwachung der Referenzprobe mit Signalen aus anderen Proben verglichen werden, um die Anzahl von Kopien jedes abgefragten genomischen Lokus für die Proben zu bestimmen.

2. Verfahren nach Anspruch 1, wobei mehr als eine DNA-Probe bereitgestellt wird und jede der DNA-Proben evaluiert wird.

3. Verfahren nach Anspruch 1, wobei die Nukleinsäure-Hybridisierungssonde eine Sammlung von Oligonukleotiden umfasst, die für einen bestimmten chromosomalen Lokus spezifisch sind.

4. Verfahren nach Anspruch 2, wobei die Referenzprobe eine bekannte Anzahl von Kopien für jeden abgefragten genomischen Lokus umfasst, und das Verfahren Vergleichen von Signalen aus der Überwachung der markierten Referenzprobe mit Signalen aus anderen Proben umfasst, um die Anzahl von Kopien jedes abgefragten genomischen Lokus für die Proben zu bestimmen.

5. Verfahren nach Anspruch 1, wobei chromosomale Amplifikation nachweisbar ist.

6. Verfahren nach Anspruch 1, wobei eine heterozygote Deletion eines Chromosoms nachweisbar ist.

7. Verfahren nach Anspruch 1, wobei eine homozygote Deletion eines Chromosoms nachweisbar ist.

8. Verfahren nach Anspruch 2, wobei der Code durch holographische Codierung oder durch eine Fluoreszenzeigenschaft bereitgestellt wird.

9. Verfahren nach Anspruch 1, in dem die DNA an das Partikel unter Verwendung eines Crosslinkers angefügt wird.

10. Verfahren nach Anspruch 1, in dem jede Probe mit dem Partikelgemisch in einem separaten Gefäß in Kontakt gebracht wird.

## Revendications

1. Procédé d'évaluation d'ADN génomique, le procédé comprenant:
la fourniture d'une pluralité d'échantillons d'ADN, dans laquelle au moins un échantillon de la pluralité d'échantillons est un échantillon de référence, avec un nombre connu de copies pour chaque locus génomique interrogé, et au moins un échantillon est un échantillon d'ADN génomique;
la fourniture d'un mélange de particules, le mélange comprenant des particules d'ensembles de particules différents, dans laquelle chaque ensemble de particules contient de nombreuses particules codées, dans laquelle chaque particule à l'intérieur d'un ensemble de particules comprend i) le même code et ii) une sonde d'hybridation d'acide nucléique pour un locus génomique particulier, dans laquelle chaque ensemble de particules différent est spécifique pour un locus génomique différent, de sorte que le mélange inclut collectivement des sondes pour une pluralité de loci génomiques différents et dans laquelle la sonde d'hybridation d'acide nucléique de chaque particule comprend un acide nucléique de BAC, dans laquelle la sonde d'hybridation de chaque particule est produite par sonification ou fragmentation de l'ADN de BAC;
le marquage de l'échantillon de référence avec un premier marqueur indirect et le marquage de l'échantillon d'ADN génomique et d'autres échantillons de la pluralité d'échantillons avec un second marqueur indirect;
la mise en contact de chacun des échantillons marqués avec une partie du mélange de particules dans des conditions d'hybridation; et
l'évaluation de l'hybridation de chacun des échantillons aux particules dans la partie respective du mélange en suivant le premier marqueur indirect ou le second marqueur indirect, ou les deux, dans laquelle les signaux du suivi de l'échantillon de référence sont comparés aux signaux d'autres échantillons pour déterminer le nombre de copies de chaque locus génomique interrogé pour les échantillons.

2. Procédé selon la revendication 1, dans lequel plus d'un échantillon d'ADN est fourni et chacun des échantillons d'ADN est évalué.

3. Procédé selon la revendication 1, dans lequel la sonde d'hybridation d'acide nucléique comprend une collection d'oligonucléotides spécifiques pour un locus chromosomique particulier.

4. Procédé selon la revendication 2, dans lequel l'échantillon de référence comprend un nombre connu de copies pour chaque locus génomique interrogé, et le procédé comprend la comparaison des signaux du suivi de l'échantillon de référence marqué avec les signaux des autres échantillons pour déterminer le nombre de copies de chaque locus génomique interrogé pour les échantillons.

5. Procédé selon la revendication 1, dans lequel une amplification chromosomique est détectable.

6. Procédé selon la revendication 1, dans lequel une délétion hétérozygote d'un chromosome est détectable.

7. Procédé selon la revendication 1, dans lequel une délétion homozygote d'un chromosome est détectable.

8. Procédé selon la revendication 2, dans lequel le code est fourni par codage holographique ou par une propriété de fluorescence.

9. Procédé selon la revendication 1, dans lequel l'ADN est attaché à la particule en utilisant un agent de réticulation.

10. Procédé selon la revendication 1, dans lequel chaque échantillon est mis en contact avec le mélange de particules dans un récipient séparé.


### SEQUENCE LISTING

<110> PerkinElmer LAS, Inc.
<120> COMPARATIVE GENOMIC HYBRIDIZATION ON
   ENCODED MULTIPLEX PARTICLES
<130> 10296-075WO1
<140> PCT/US2006/048801
   <141> 2006-12-22
<150> US 60/753,584
   <151> 2005-12-23
<150> US 60/753,822
   <151> 2005-12-23
<150> US 60/765,311
   <151> 2006-02-03
<150> US 60/765,355
   <151> 2006-02-03
<160> 33
<170> FastSEQ for Windows Version 4.0
<210> 1
   <211> 69
   <212> DNA
   <213> Artificial Sequence
<220>
   <223> Synthetically generated oligonucleotide
<400> 1
   tgagacacca taaagaagtt ggtctgccct aacagtgtgt ctacaagctt gtaaagatgt 60
   tggccttga                                                          69
<210> 2
   <211> 70
   <212> DNA
   <213> Artificial Sequence
<220>
   <223> Synthetically generated oligonucleotide
<400> 2
   tcacctgatt cttccaatga gaattccgta gcaactcctc ctccagagga acaagggcaa 60
   ggtgatgccc                                                         70
<210> 3
   <211> 69
   <212> DNA
   <213> Artificial Sequence
<220>
   <223> Synthetically generated oligonucleotide
<400> 3
   ttgaaccaag tgtttttaca tgacaagttc tctgaggatg gttctacagt tgggattttg 60
   gccatcatc                                                          69
<210> 4
   <211> 69
   <212> DNA
   <213> Artificial Sequence
<220>
   <223> Synthetically generated oligonucleotide
<400> 4
   atggggcagc agttagaggg ttgtgctctt tctagtgtgg gatagtttgc aagatgatat 60
   gttgtagcc                                                          69
<210> 5
   <211> 70
   <212> DNA
   <213> Artificial Sequence
<220>
   <223> Synthetically generated oligonucleotide
<400> 5
   tgtgcgggtt aatacaacaa actgtcacaa gtgtttgttg tccgggacgt acattttcgc 60
   ggtcctgcta                                                        70
<210> 6
   <211> 70
   <212> DNA
   <213> Artificial Sequence
<220>
   <223> Synthetically generated oligonucleotide
<400> 6
   ccaacacccc agctccgctg ctgccgccac cgcagtgctc tctagtcgcc attggttacc 60
   taaactttcc                                                        70
<210> 7
   <211> 70
   <212> DNA
   <213> Artificial Sequence
<220>
   <223> Synthetically generated oligonucleotide
<400> 7
   aggtgtgagc caccatgccc ggtaaacttt taaaaatgta agcaaaatta cagtatgtaa 60
   aacacacatt                                                        70
<210> 8
   <211> 70
   <212> DNA
   <213> Artificial Sequence
<220>
   <223> Synthetically generated oligonucleotide
<400> 8
   tcatgcagcc tgcaccagcg ccgggtcgga gagtcagagg ccaccctgag atggaccgag 60
   atcttcagtt                                                        70
<210> 9
   <211> 70
   <212> DNA
   <213> Artificial Sequence
<220>
   <223> Synthetically generated oligonucleotide
<400> 9
   ttgggagggg taatagtgaa gtgtttttcc actaaattac ttttttctaa tcagtgtgaa 60
   gtgacacagg                                                        70
<210> 10
   <211> 70
   <212> DNA
   <213> Artificial Sequence
<220>
   <223> Synthetically generated oligonucleotide
<400> 10
   tcacacccac tgctggacag tgtgagtcag gggcagcctg gactgatgcc atggcatttc 60
   tgcttgctaa                                                        70
<210> 11
   <211> 69
   <212> DNA
   <213> Artificial Sequence
<220>
   <223> Synthetically generated oligonucleotide
<400> 11
   cttgagtttc tatactttta agaagagctc tttgttcctg ggggaggggg gcagggggtg 60
   aattttact                                                        69
<210> 12
   <211> 69
   <212> DNA
   <213> Artificial Sequence
<220>
   <223> Synthetically generated oligonucleotide
<400> 12
   acaacctcgg gaacgttcat tccccgctga agcttctcac cagcatggcc atctcggtgg 60
   tgtgcttct                                                         69
<210> 13
   <211> 69
   <212> DNA
   <213> Artificial Sequence
<220>
   <223> Synthetically generated oligonucleotide
<400> 13
   tccttaaagg acagactgaa tggtattcga cgagtcctgg ccttcatatc ccgaaaccaa 60
   aactagtga 69
<210> 14
   <211> 69
   <212> DNA
   <213> Artificial Sequence
<220>
   <223> Synthetically generated oligonucleotide
<400> 14
   cgtgcctatg gcgggaccac gctcggagcc tgcctcttct gcgactgtta ctttttcttt 60
   gcgggatgg 69
<210> 15
   <211> 69
   <212> DNA
   <213> Artificial Sequence
<220>
   <223> Synthetically generated oligonucleotide
<400> 15
   ctgtctctct gcgcttggaa caccttcccc tgtgtactac tggcataaac ttgagggaag 60
   agacatcgt 69
<210> 16
   <211> 69
   <212> DNA
   <213> Artificial Sequence
<220>
   <223> Synthetically generated oligonucleotide
<400> 16
   gtccgatgcc agcctccaat ttcgctgcgg caccggcttt ctcgcactat atggggtatc 60
   ctcatatgc 69
<210> 17
   <211> 69
   <212> DNA
   <213> Artificial Sequence
<220>
   <223> Synthetically generated oligonucleotide
<400> 17
   aagagagtca caggtacccc aaggagtaga tgccagggtc ctaagttgaa aatgatgtcg 60
   attgggggc                                                         69
<210> 18
   <211> 69
   <212> DNA
   <213> Artificial Sequence
<220>
   <223> Synthetically generated oligonucleotide
<400> 18
   gcaatttctg tcgccgtcct gtacactttg agctcatgag tgagtgggaa cgttcctact 60
   tcgggaaca                                                         69
<210> 19
   <211> 70
   <212> DNA
   <213> Artificial Sequence
<220>
   <223> Synthetically generated oligonucleotide
<400> 19
   agcttctttg ccaagacctt tcaaagccat tttaggctgt taggggcagt ggaggtagaa 60
   tgactccttg                                                        70
<210> 20
   <211> 70
   <212> DNA
   <213> Artificial Sequence
<220>
   <223> Synthetically generated oligonucleotide
<400> 20
   agcaacgccc gttattttca tgtggtcatt gctggggaat caaaggattc cccctttgag 60
   ggagggactt                                                        70
<210> 21
   <211> 69
   <212> DNA
   <213> Artificial Sequence
<220>
   <223> Synthetically generated oligonucleotide
<400> 21
   ttggaactag ggtcatttga aagcttcagt ctcggaacat gacctttagt ctgtggactc 60
   catttaaaa                                                         69
<210> 22
   <211> 69
   <212> DNA
   <213> Artificial Sequence
<220>
   <223> Synthetically generated oligonucleotide
<400> 22
   ccatggacca cgcaggaagg gcctacagcc catttctcca tacgcactgg tatgtgtgga 60
   tgatgctgc 69
<210> 23
   <211> 69
   <212> DNA
   <213> Artificial Sequence
<220>
   <223> Synthetically generated oligonucleotide
<400> 23
   ctggcacagt gtaatccagg ggtgtagatg ggggaactgt gaatacttga actctgttcc 60
   cccaccctc 69
<210> 24
   <211> 69
   <212> DNA
   <213> Artificial Sequence
<220>
   <223> Synthetically generated oligonucleotide
<400> 24
   ggcttagggt cggaacccaa gcttagaact ttaagcaaca agaccaccac ttcgaaacct 60
   gggattcag 69
<210> 25
   <211> 69
   <212> DNA
   <213> Artificial Sequence
<220>
   <223> Synthetically generated oligonucleotide
<400> 25
   tacaccataa agcggcacaa gtggtcagtc ctgaagagtc ggaagctggc atatcggccg 60
   cccaagtga 69
<210> 26
   <211> 69
   <212> DNA
   <213> Artificial Sequence
<220>
   <223> Synthetically generated oligonucleotide
<400> 26
   gatttcttca gggctgctgg gggcaactgg ccatttgcca attttcctac tctcacactg 60
   gttctcaat                                                         69
<210> 27
   <211> 69
   <212> DNA
   <213> Artificial Sequence
<220>
   <223> Synthetically generated oligonucleotide
<400> 27
   ctgcgggtaa agtgtgagag cttgccatcc agctcacgaa gacagagtta ttaaaccatt 60
   acaaatctc                                                         69
<210> 28
   <211> 69
   <212> DNA
   <213> Artificial Sequence
<220>
   <223> Synthetically generated oligonucleotide
<400> 28
   aactggagtc tgacttggtt cgttagtgga ttacttctga gcttgcaaca tagctcactg 60
   aagagctgt                                                         69
<210> 29
   <211> 69
   <212> DNA
   <213> Artificial Sequence
<220>
   <223> Synthetically generated oligonucleotide
<400> 29
   cgagcacgga acaatggggc cttcagctgg agctgtggac ttttgtacat acactaaaat 60
   tctgaagtt                                                         69
<210> 30
   <211> 69
   <212> DNA
   <213> Artificial Sequence
<220>
   <223> Synthetically generated oligonucleotide
<400> 30
   gcagtgctga gtcaggattt ggggccggct ctcttgggtc tgtccccttt tcccaggtac 60
   tgccttaca                                                         69
<210> 31
   <211> 69
   <212> DNA
   <213> Artificial Sequence
<220>
   <223> Synthetically generated oligonucleotide
<400> 31
   tcttggtgac tgtcccaccg gagcctcccc ctcagatgat ctctccacgg tagcacttga 60
   ccttttcga                                                         69
<210> 32
   <211> 69
   <212> DNA
   <213> Artificial Sequence
<220>
   <223> Synthetically generated oligonucleotide
<400> 32
   ttttccgagt ggcagctgac atgttttctg acggcaactt caactggggc cgggttgtcg 60
   cccttttct                                                         69
<210> 33
   <211> 69
   <212> DNA
   <213> Artificial Sequence
<220>
   <223> Synthetically generated oligonucleotide
<400> 33
   tgtaccttgg gtggcacttg ttatgctatc ctgtgctagc cgtttgtgcc tcgtctcgct 60
   gttagattg                                                         69